# EUROPEAN PATENT APPLICATION

(11) **EP 2 392 268 A1**
(43) Date of publication of application: **07.12.2011**
(21) Application number: 10165151.1
(22) Date of filing: 07.06.2010
(51) Int. Cl.: A61B 17/122, A61B 17/128, A61B 17/00

(54) **Improved vascular clamp structure**

(71) Applicant: Top-Bound Enterprise Co. Ltd., Wugu Township, Taipei County 24891 (TW); Hong-So, Kao, Da'an Dist., Taipei City 10642 (TW)
(72) Inventor: Yeh, Yen-Ming, Banqiao City, Taipei County 22041 (TW)
(74) Representative: Chaillot, Geneviève

(57) **Abstract**

An improved vascular clamp structure is comprised of a clip body and two clipping pieces, all of which constitute a vascular clamp used cooperatively for medical clamping pliers. The clip body is arranged with a bridge part, and from two ends of which a clipping part extends respectively and making one side of the clip body forming an opening; the clipping piece is arranged with a clipping body with a fitting slot. The fitting slot and the clipping part can be assembled together. Both adjacent faces of the clipping bodies are respectively arranged with a groove and at least one male or female fastener inter-buckled together. Wherein, the clip body is set as a memory metal. By arranging clipping pieces, the vascular clamp of the present invention enhances the frictional areas of inter-contact. Arranging male and female fasteners at the adjacent faces of clipping pieces also enhances the clipping force, and effectively improves the slipping drawback of traditional vascular clamp. In addition, the vascular clamp is made of memory metal; by the separation of male and female fasteners, the vascular clamp further possesses the function of dismantlement.

## Description

### FIELD OF THE INVENTION

The present invention in general relates to a vascular clamp cooperatively used for medical pliers, in particular, to an improved vascular clamp structure with a clip body, two ends of which have clipping pieces capable of clipping enhancement.

### Description of Prior Art

### BACKGROUND OF THE INVENTION

In general surgery medical operation, it needs to incise or ligate blood vessels within an organ or tissue. Since organs and tissues of human bodies are not completely identical, and each person's individual constitutions are not all the same, so vascular ligations required in surgery are also different.

General vascular ligation methods are divided into three types: the first method is to carry suture ligation to the wall of blood vessels; the second one is to carry electric sintering ligation on the vascular wall with laser, high frequency electric knife or other plasma of high amount of energy; and, the last method is to use a medical clamp to clamp the bleeding blood vessel wall for executing an outside ligation.

In terms of the application method of hemostat, a hemostat is usually designed as two leg parts opposite to each other and capable of being open. After suppressed by suppression units of a medical plier, two leg parts will be closed. Common hemostats are made of materials of metal or polymer, such that the hemostats can have sufficient strength to make the clamps maintain clipping and closing positions when two leg parts are suppressed together.

However, traditional hemostats are all comprised of a single clip body. This kind of design results in small contacting area between the two leg parts of the clamp and the vessel, causing an occurrence of slipping phenomenon, due to insufficient friction exerting between the vascular clamp and vessel. Furthermore, it is difficult to separate the two leg parts after the hemostat of a traditional single clip body has clamped something. Thus, it is uneasy to dismantle the hemostat, and the current hemostat still has the necessity for improvement and innovation. Accordingly, the present invention designs a vascular ligation instrument that has a larger friction area and has the capability of being easily dismantled.

Accordingly, after a substantially devoted study, in cooperation with the application of relative academic principles, the inventor has finally proposed the present invention designed reasonably to possess the capability to improve the drawbacks of the prior arts significantly.

### SUMMARY OF THE INVENTION

The main objective of the present invention is to provide an improved vascular clamp structure. Two ends of the vascular clamp are respectively equipped with a clipping piece, whereby the friction area between the inter-contacting parts of the vascular clamp can be increased. At adjacent faces between two clipping pieces, there is a set of male and female fasteners inter-assembled to each other, effectively enhancing the clipping force of the vascular clamp, and improving the drawback of easy slippage of traditional vascular clamps.

Another objective of the present invention is to provide a vascular clamp made of metal with restoration effectiveness. When the male and female fasteners are separated by dislocation under assembly status, the vascular clamp can be restored to original formation status from clipping status, making the vascular clamp have a dismantlement function.

In order to achieve aforementioned objectives, an improved vascular clamp of the present invention can match the usage of a medical clamping plier, including a clip body, a first clipping piece, and second clipping piece. The clip body has a bridge part with an inflecting point. Two ends of the bridge part respectively extend a first clipping part and a second clipping part, making one side of the clip body form an accommodation opening fitted to a vessel;

The first clipping piece is arranged as a first clipping body with a first fitting slot and is assembled fixedly by the first fitting slot and the first clipping part of the clip body. The first clipping body is arranged as a first groove and at least one male fastener at a face adjacent to a second clamping piece.

The second clipping pieces is arranged as a second clipping body with a second fitting slot and is assembled fixedly by the second fitting slot and the second clipping part of the clip body.

The second clipping body is arranged as a second groove and at least one female fastener matching the male fastener at the face adjacent to the first clipping piece.

Wherein, the clip body can be set as a memory metal with restoration effectiveness. When the male and female fasteners of the first and second clipping pieces are separated, the memory metal of the clip body will restore the bridge part and two clipping parts to original formation pattern.

The first, second clipping parts and the first, second fitting slots are configured as wedged dovetails assembled and matched by corresponding to each other. Wherein, the wedged dovetails are to set the inside faces of the first, second fitting slots as two oblique walls and set the outside faces of the first, second clipping part as two bevel edges corresponding to each other. Besides, the bottom sides of two oblique walls can be additionally arranged with a ring-guiding slot respectively to be inter-wedged fixedly with a pair of corresponding guiding rails arranged at corresponding bottom sides of two bevel edges.

In a preferable embodiment, the first groove is set as a convex wedge block projected from the surface of the first clipping body, while the second groove is set as a concave wedge slot recessed in the surface of the second clipping body. In this case, the convex wedge block is arranged as corresponding to the configuration of the concave wedge slot. The convex wedge block is arranged with a plurality of longitudinal ribs interspaced separately. Between the longitudinal ribs, a plurality of oblique ribs is arranged as being interspaced with the longitudinal ribs. The concave wedge slots are arranged with a plurality of longitudinal slots corresponding to the longitudinal ribs and a plurality of oblique slots corresponding to the oblique ribs.

In another preferable embodiment, the said first groove is comprised of a plurality of flat cone convex patterns projected from the surface of the first clipping body and adjacently interspaced to each other, while the second groove is also comprised of a plurality of flat cone convex patterns projected from the surface of the second clipping body and adjacently interspaced to each other.

The male fastener is a diverging insetting piece, while the female fastener is an insetting hole with a converging structure therein. The insetting piece is comprised of two insetting ribs extending and diverging to the outside from the connecting side of the clipping body. An elastic space is formed between the insetting ribs. The converging structure is configured by forming a projection block respectively at sides of the opening of the insetting hole. A wedge block is formed at a center of the converging structure, making two sides of the wedge block respectively forming a hole capable of being passed by the insetting rib. The male fastener can be arranged at a center of the bottom part of the first clipping body of the first clipping piece, while the female fastener can be arranged at a center of the bottom part of the second clipping body of the second clipping piece.

The characteristic of the invention is that two clipping pieces are respectively arranged at two ends of the clip body of the vascular clamp to increase the friction area between the inter-contacted contacting parts of the vascular clamp. The adjacent faces of two clipping pieces are arranged with a set of corresponding grooves and at least one set of male and female fasteners inter-assembled to each other, effectively enhancing the forces of longitudinally and vertically stabilizing the vessel.

Moreover, the vascular clamp is made of memory metal capable of restoration effectiveness. Through the dislocation separation of the male and female pieces under an assembly status, the vascular clamp can be restored to original formation status from clipping status, such that the vascular clamp possesses the function of dismantlement.

### BRIEF DESCRIPTION OF DRAWING

The features of the invention believed to be novel are set forth with particularity in the appended claims. The invention itself, however, may be best understood by reference to the following detailed description, which describes a number of embodiments of the invention, taken in conjunction with the accompanying drawings, in which:
Fig. 1 is a perspective view of an improved vascular clamp structure according to a preferable embodiment of the present invention;
Fig. 2 is an explosive and separated view of a clip body and two clipping pieces of the vascular clamp in Fig. 1;
Fig. 3A to Fig. 3C are structural illustrations of the clip body of an vascular clamp according to different preferable embodiments of the present invention;
Fig. 4 is a longitudinally sectioned and separated view of a clip body and two clipping pieces of the vascular clamp in Fig. 2;
Fig. 5 is an illustration showing a suppressed (closed) status and a dislocated (open) status of a vascular clamp of the present invention;
Fig. 6A to Fig. 6B are sectional illustrations of the clip body and the clipping piece set as wedged dovetails in a vascular clamp of the present invention;
Fig. 7 is an illustration showing the surface grooves of two clipping pieces of a vascular clamp according to a preferable embodiment of the present invention;
Fig. 8 is an illustration showing the surface grooves of two clipping pieces of a vascular clamp according to another preferable embodiment of the present invention;
Fig. 9 is a bottom view of the male and female fasteners arranged at the bottom faces of two clipping pieces of a vascular clamp of the present invention;
Fig. 10 is a usage illustration of an endoscopic surgery clamping plier matched with a vascular clamp of the present invention;
Fig. 11 is a usage illustration of an open surgical clamping plier matched with a vascular clamp of the present invention; and
Fig. 12 is an illustration showing a suppressed vascular clamp according to the present invention when clamping a vessel.

### DETAILED DESCRIPTION OF THE PREFERRED EMBODIMENTS

In cooperation with attached drawings, the technical contents and detailed description of the present invention are described thereinafter according to a number of preferable embodiments, not used to limit its executing scope. Any equivalent variation and modification made according to appended claims is all covered by the claims claimed by the present invention.

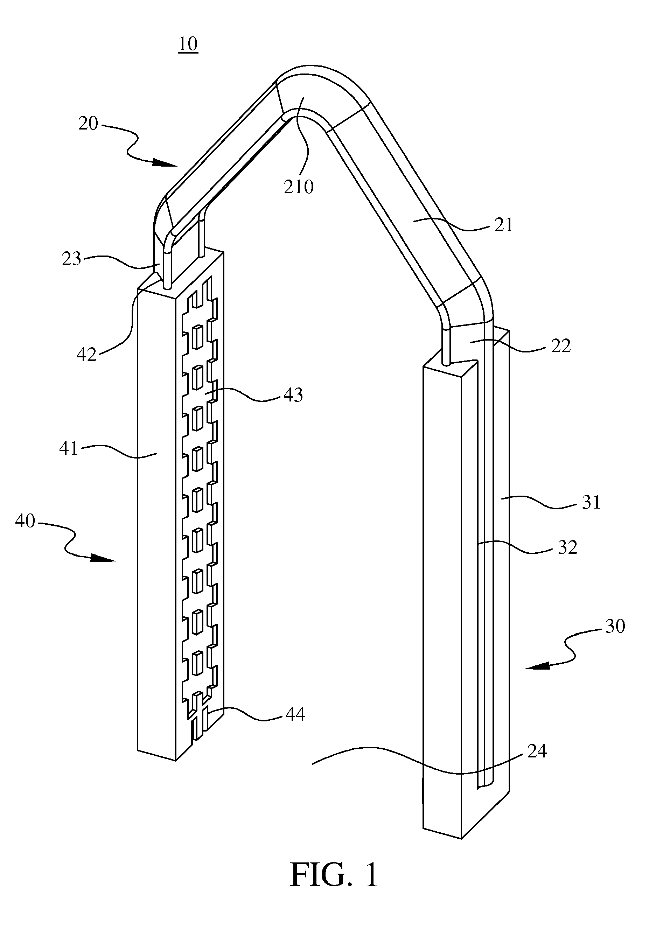

First of all, please refer to Fig. 1 and Fig. 10 to Fig. 12. As shown in these figures, the vascular clamp 10 of the invention is comprised of a clip body 20, a first clipping piece 30 connected to one end of the clip body 20 and a second clipping piece 40 connected to another end of the clip body 20. During an operation, the vascular clamp 10 is matched with the medical clamping plier 50 and suppressed to deform to ligate a vessel.

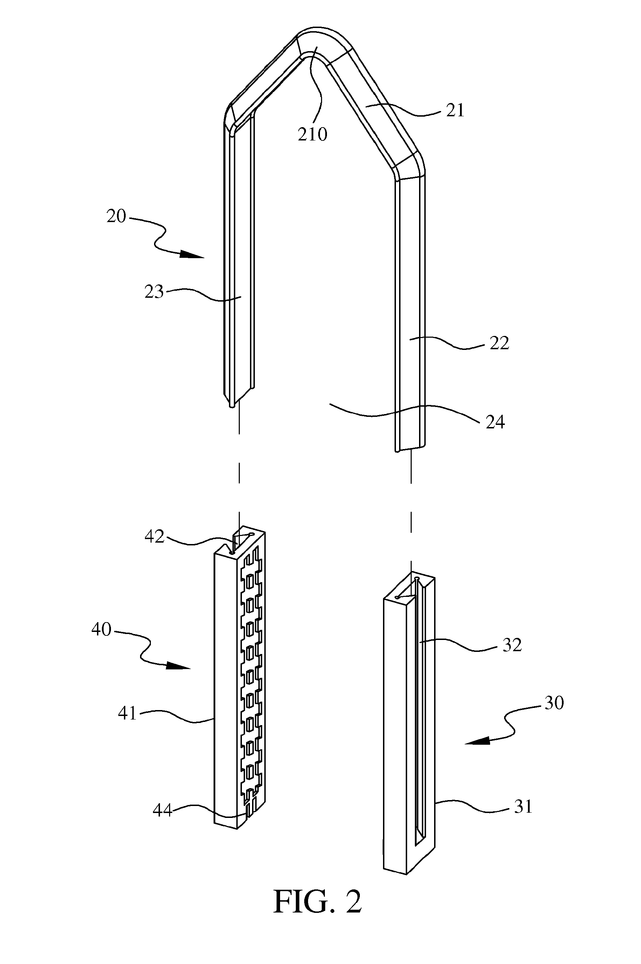

Secondly, please refer to Fig. 2. As shown in this figure, the clip body 20 of the vascular clamp 10 is arranged with a bridge part 21. The bridge part 21 is configured as a V-shaped structure with an inflecting point 210. Two ends of the bridge part 21 respectively extend a first clipping part 22 shown vertically and a second clipping part 23 also shown vertically and parallel to the first clipping part 22. The bridge part 21, the first clipping part 22 and the second clipping part 23 jointly form an opening 24 for setting a vessel into one side of the clip body 20. However, this description is only an example for the convenience of illustration, and does not restrict the structural pattern of the clip body 20.

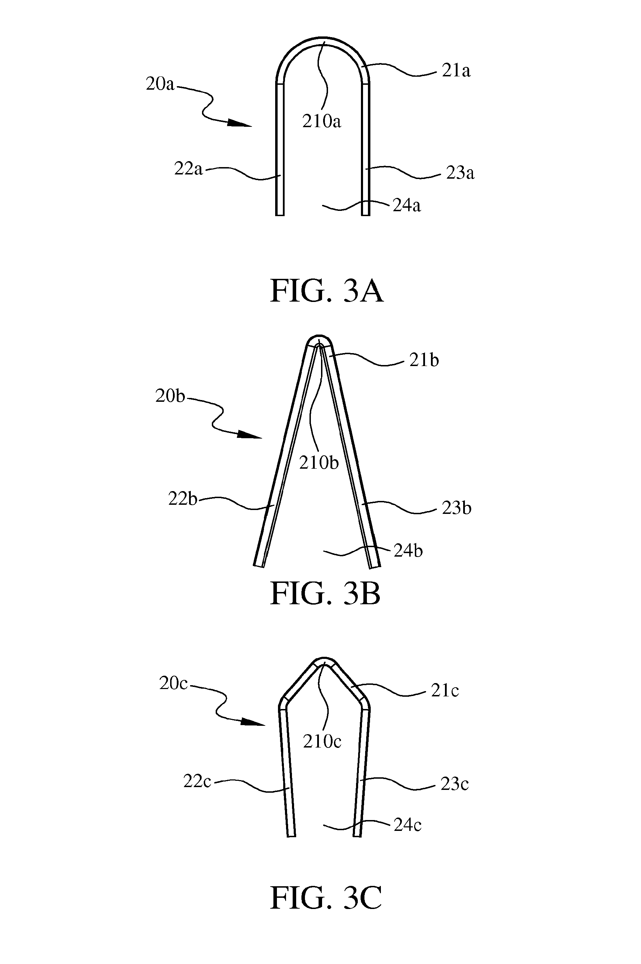

Furthermore, please refer to Fig. 3A. As shown in this figure, the bridge part 21a of the clip body 20a can also be set as a curved structure with an inflecting point 210a. Then, two ends of the bridge part 21a are respectively arranged and extended as a first clipping part 22a and a second clipping part 23a. And the clipping part 22a and 23a are shown vertically and parallel to each other. In addition, the opening 24a is formed between the first clipping part 22a and the second clipping part 23a. By so doing, a structure of clip body 20a with different pattern can be configured.

Please refer to Fig. 3B. As shown in this figure, the bridge part 21b of the clip body 20b can also be set as a V-shaped structure with an inflecting point 210b. Then, two ends of the bridge part 21b are respectively arranged as a first clipping part 22b and a second clipping part 23, that both horizontally extend outwardly. The clip body 20b of this embodiment can form an opening 24b with a larger aperture.

Please refer to Fig. 3C. As shown in this figure, the bridge part 21c of the clip body 20c can also be set as a V-shaped structure with an inflecting point 210c. Then, two ends of the bridge part 21c are respectively arranged as a first clipping part 22c and a second clipping part 23c that both incline inward to form an opening 24c with a smaller aperture.

It is known that the shapes of the clip bodies 20, 20a, 20b, 20c of the present invention are configured according to the needs of operation, or the sizes of the apertures of the openings 24, 24a, 24b, 24c. Regarding the structural variations of other kinds of clip bodies 20, 20a, 20b, 20c, they can all be simply obtained from the aforementioned embodiments disclosed in Fig. 3A to Fig. 3C without the need of further description.

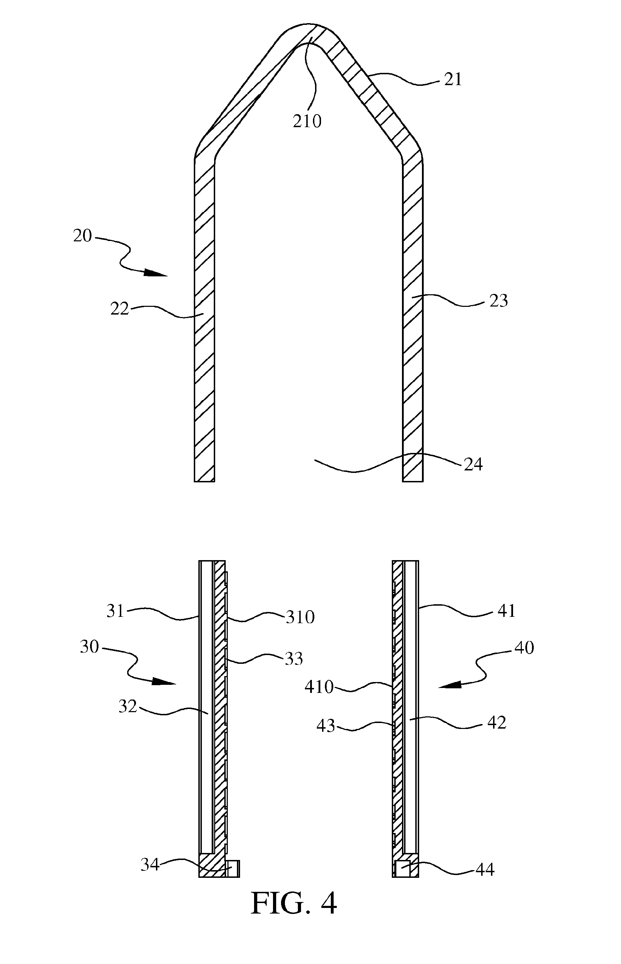

Please refer to Fig. 4. As shown in this figure, the first clipping piece 30 is equipped with a first clipping body 31, and a first fitting slot 32 is set at the top of the first clipping body 31. The first clipping part 22 of the clip body 20 is assembled fixedly by the first fitting slot 32, whereby the first clipping piece 30 is firmly fixed to one end of the clip body 20. At the face 310 of the clipping body 31 and adjacent to the second clipping piece 40, and equipped with a first groove 33 and at least one male fastener 34.

The second clipping piece 40 is equipped with a second clipping body 41, and a second fitting slot 42 is arranged at the top of the second clipping body 41. The second clipping part 23 of the clip body 20 is assembled fixedly by the second fitting slot 42, whereby the second clipping piece 40 is firmly fixed to another end of the clip body 20. At one face 410 adjacent to the first clipping piece 30, the second clipping body 41 is equipped with a second groove 43, and at least one female fastener 44 matches the male fastener 34 to possess buckling and positioning functions.

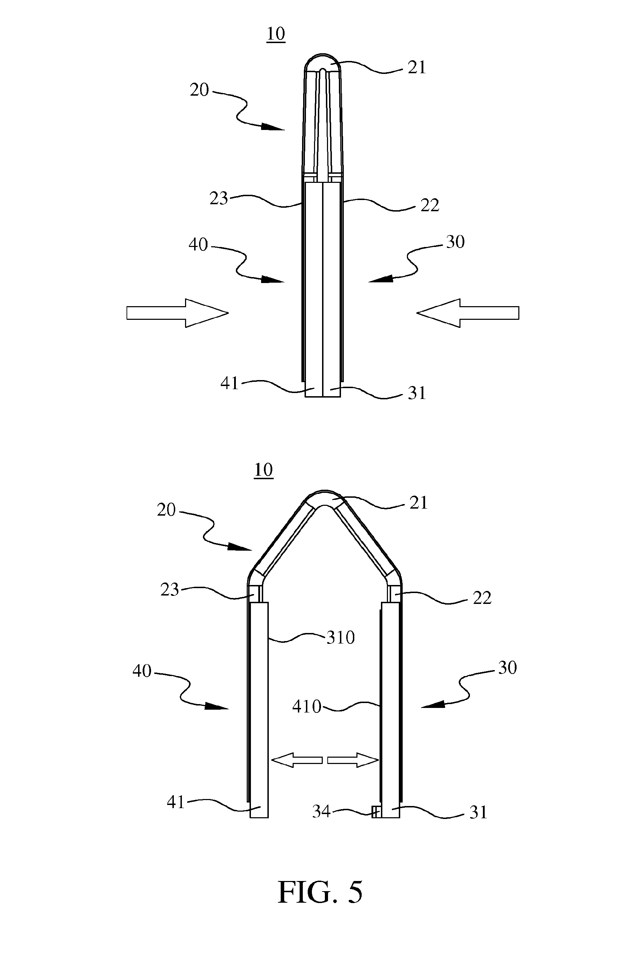

Please refer to Fig. 5. As shown in this figure, the clip body 20 of the invention can be set as a memory metal with the effectiveness of restoration. Therefore, when the first and second clipping pieces 30, 40 and the male and female fasteners 34, 44 are dislocated and separated under an assembly status, the material of the memory metal of the clip body 20 can restore the bridge part 21, the first clipping part 22, and the second clipping part 23 to an original pre-configured pattern. This kind of innovative design can make the vascular clamp 10 possess both the clamping and dismantling functions.

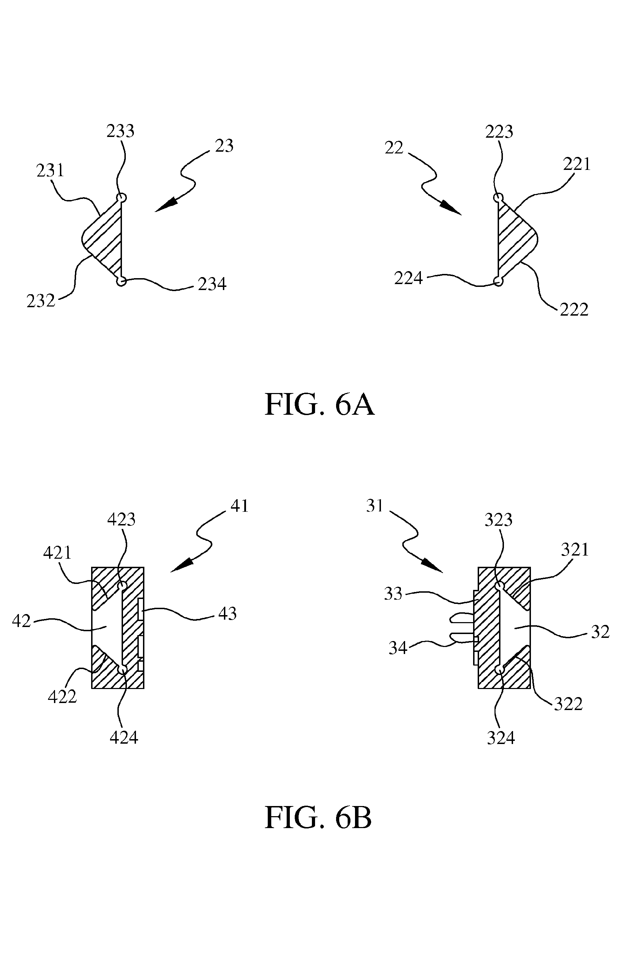

Please refer to Fig. 6A and Fig. 6B. As shown in these two figures, in a preferable embodiment, the first and second clipping parts 22, 23 of the clip body 20, the first fitting slot 32 of the first clipping piece 30 and the second fitting slot 42 of the second clipping piece 40 can be matched to be assembled together by being configured as wedged dovetails corresponding to one another.

The wedged dovetails are made by setting the inside faces of both the first fitting slot 32 and the second fitting slot 42 respectively as two oblique walls 321, 322, 421, 422 diverged to the inside from the surfaces of the clipping pieces. On the other hand, the outside faces of the first clipping part 22 and the second clipping part 23 are arranged as two bevel edges 221, 222, 231, 232 wedged correspondingly with the oblique walls 321, 322, 421, 422. In addition, the ring-guiding slots 323, 324, 423, 424 are separately arranged at bottom sides of the two oblique walls 321, 322, 421, 422, while the corresponding positions of the bottom sides of the two bevel edges 221, 222, 231, 232 are separately arranged to correspond with guiding rails 223, 224, 233, 234, such that the slots and rails can be wedged together fixedly. With the close fit between the structures of the clip body 20 and the two clipping pieces 30, 40, the clip body 20 is firmly combined with the first clipping piece 30 and the second clipping piece 40.

This embodiment is only one kind of preferable structure for the convenience of description. The first and second clipping parts 22, 23 and the first and second fitting slots 32, 42 can be set as fitting structures of any other geometrical shapes that can be inter-matched together.

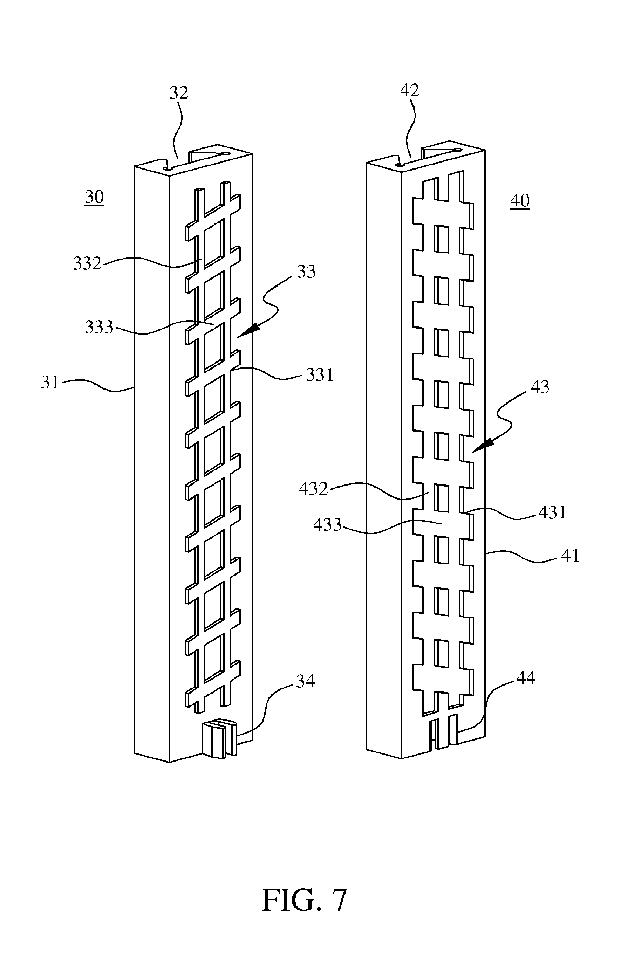

Please refer to Fig. 7. As shown in this figure, the first groove 33 of the first clipping body 31 is arranged as a convex wedge block 331 projected from the surface of the first clipping body 31, and the second groove 43 of the second clipping body 41 is arranged as a concave wedge slot 431 recessed in the surface of the second clipping body 41. The shapes of the convex wedge block 331 and the concave wedge slot 431 are inter-corresponded to each other such that, when the first clipping piece 30 and the second clipping piece 40 are fixed together by being inter-contacted to each other, more effective anti-slipping friction can be generated.

This embodiment contains the convex wedge block 331 with a plurality of longitudinal ribs 332 interspaced separately. And a plurality of oblique ribs 333 are arranged as interspaced separately among and interlacing with the longitudinal ribs 332. The concave wedge slot 431 is composed of longitudinal notches 432 corresponding to the longitudinal ribs 332 and oblique slots 433 corresponding to the oblique ribs 333. The said description is one of the embodiment of the convex wedge block 331 and the concave wedge slot 431. The convex wedge block 331 and the concave wedge slot 431 can be arranged as ribs or notches with only one of horizontal, vertical or oblique directions. Or, the convex wedge block 331 and the concave wedge slot 431 can be arranged as ribs or notches staggered in three or more directions.

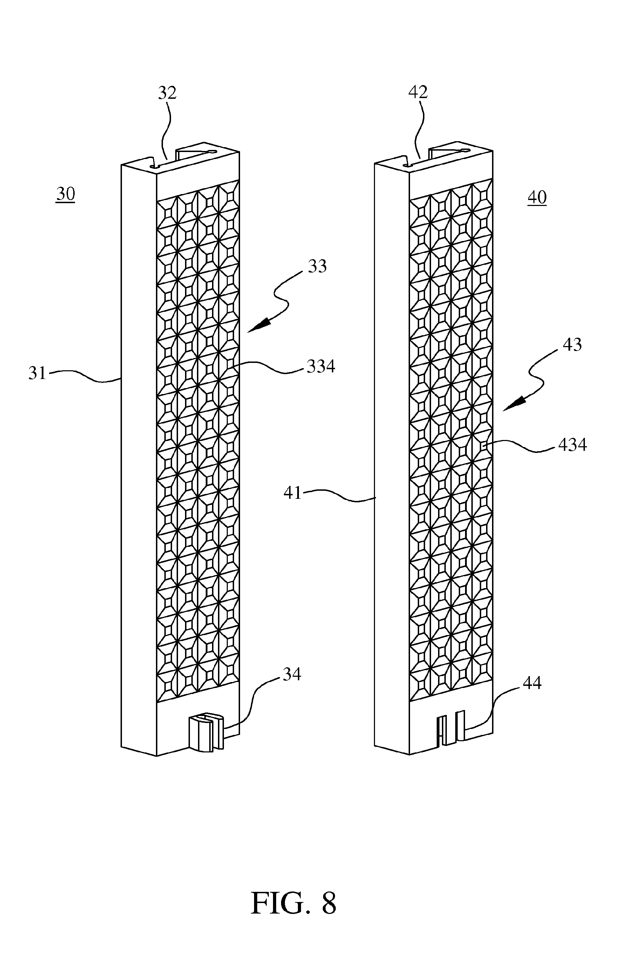

As shown in Fig. 8, in another embodiment, the first groove 33 of the first clipping body 31 can also be arranged with a plurality of flat cone convex patterns 334 projected from the surface of the first clipping body 31 and adjacently laid over the entire adjacent face. On the other hand, the second groove 43 can be arranged as a plurality of corresponding flat cone convex patterns 434 projected from the surface of the second clipping body 41 and adjacently laid over the entire adjacent face 410. Through the staggered clamping manner, it can also make vascular clamp 10 generate more effective anti-slipping friction.

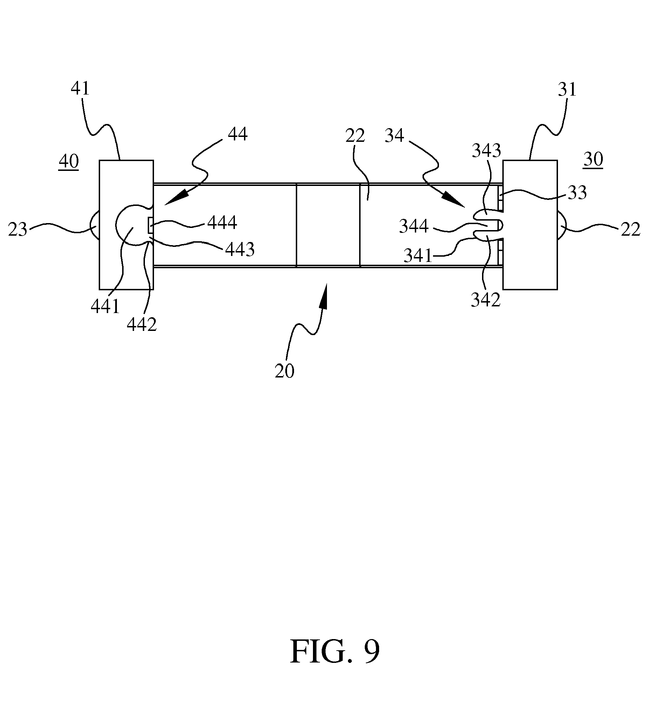

As shown in Fig. 9, in a preferable embodiment, the male fastener 34 is arranged at a center of the bottom part of the first clipping body 31 of the first clipping piece 30, while the female fastener 44 can be arranged at a center of the bottom part of the second clipping body 41 of the second clipping piece 40. By means of corresponding pair of male and female fasteners 34, 44, the invention increases the clipping force to clip object. If the vascular clamp 10 needs stronger clipping force, then the number of sets of the male and female fasteners 34, 44 can be increased to meet the needed clipping force.

The male fastener 34 is a diverging and insetting piece 341 projected outwardly, while the female fastener 44 is an insetting hole 441 with a converging structure therein. Wherein, the insetting piece 341 is comprised of two insetting ribs 342, 343 extending and diverging outwardly to a connecting side of the first clipping body 31, and an elastic space 344 is formed between the two insetting ribs 342, 343, while the converging structure 443 is configured by forming a projection block 442 respectively at two sides of the opening of the insetting hole 441, and a wedge block 444 is formed at a center of the converging structure 443, making two sides of the wedge block 444 respectively formed a hole capable of being passed by the insetting ribs 342, 343.

Similarly, this description is only one kind of executable structure of the male and female fasteners 34, 44. Other kinds of connection structures are also acceptable, as long as the same effectiveness can be achieved. Since there are many categories and patterns for this kind of connection structure, further description is not presented here.

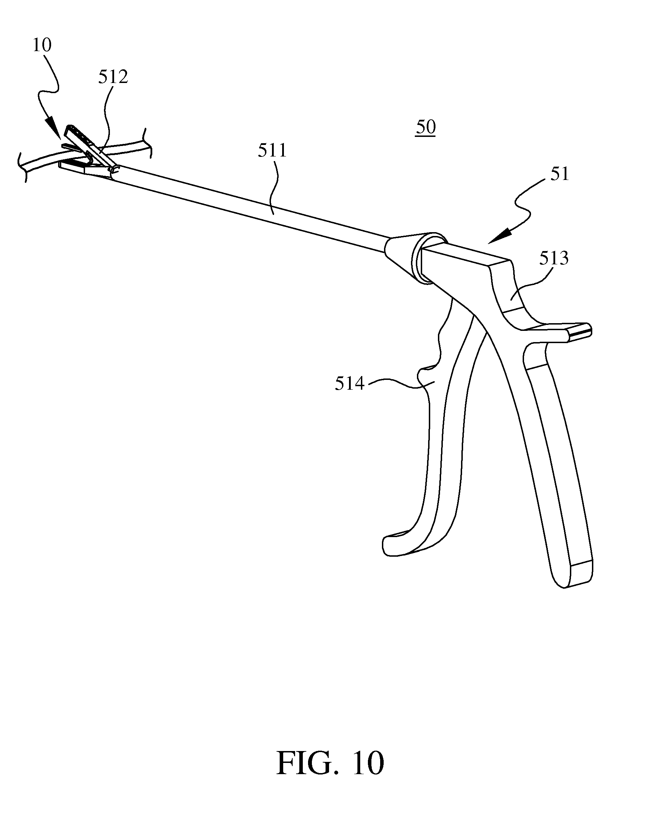

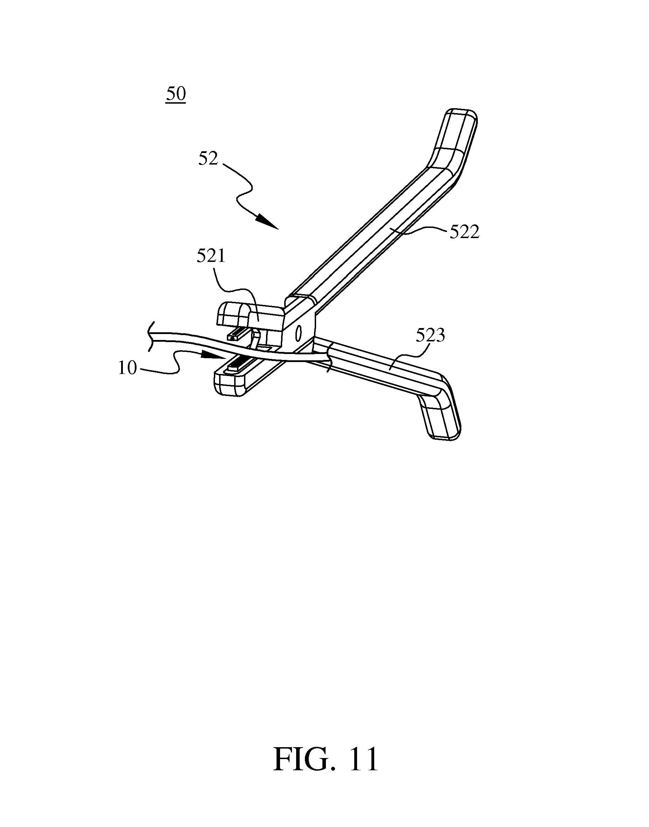

Please refer to Fig. 10 and Fig. 11. A vascular clamp 10 of the invention matches the usage of a medical clamping plier 50. This medical clamping plier 50 can be a clamping plier for endoscopic surgery 51 (e.g. Fig. 10) or a clamping plier for open surgery 52 (e.g. Fig. 11).

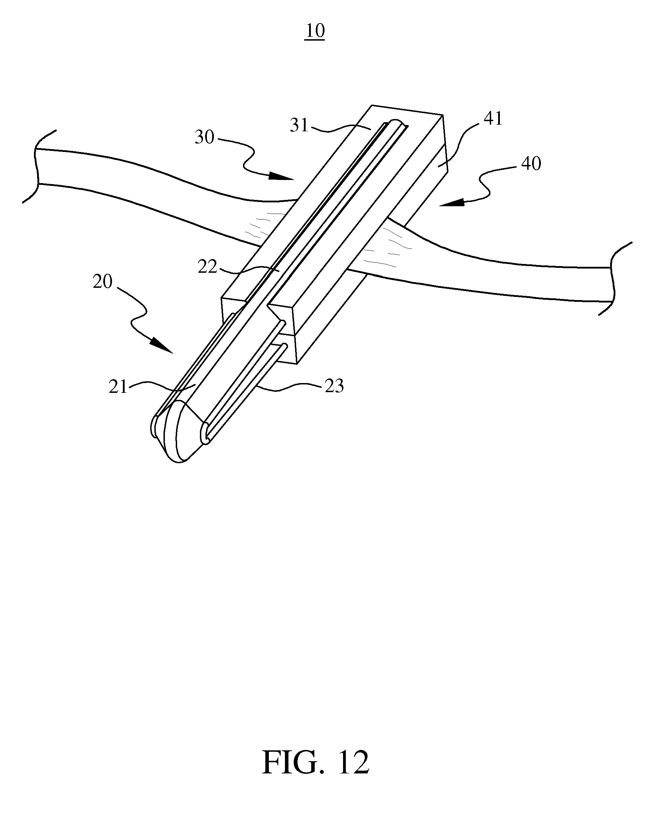

If applied in a clamping plier for endoscopic surgery 51, it needs to place the vascular 10 onto a clamping part 512 at the end part of a transmission tube 511 of the clamping plier for endoscopic surgery 51. Next, the clamping part 512 is placed onto the part needed the surgical ligation. Then, by pressing down a start pull handle 514 of the gripping part 513, the clamping part 512 suppresses the outside of the vascular clamp 10. As shown in Fig. 12, the vascular clamp 10 will be contracted inwardly to become a long-stripe shape, whereby the part needing ligation is clamped.

If applied in a clamping plier for open surgery 52, it needs to place the vascular 10 onto another clamping part 521 of the clamping plier for open surgery 52. Then, the clamping part 521 is moved to a ligation part needing an open surgery. By pressing down two handle-gripping parts 522, 523, the clamping part 521 suppresses the outside of the vascular clamp 10. In a same way, the vascular clamp 10 will be contracted inwardly to become a long-stripe shape, whereby the part needing ligation is clamped.

In summary, the improved vascular clamp structure of the invention is to respectively arrange a clipping piece at two ends of the clip body. By so doing, the friction area between the inter-contacting parts of vascular clamp increases. On the other hand, a set of corresponding grooves and at least a set of male and female fasteners are arranged at two adjacent faces of the two clipping pieces, effectively enhancing the horizontal clipping and vertical stabilizing forces of the vascular clamp. In addition, the vascular clamp is made of memory metal possessing the effectiveness of restoration. When the male and female fasteners are separated and dislocated under an assembly status, the vascular clamp will restore to original formation status from a clipping status. Therefore, the vascular clamp has a dismantling function.

Through the constitution of aforementioned assemblies, an improved vascular clamp structure according to the present invention is thus obtained.

Summarizing aforementioned description, the improved vascular clamp structure is an indispensable device and structure for a medical surgery and operation indeed, which may positively reach the expected usage objective for solving the drawbacks of the prior arts, and which extremely possesses the innovation and progressiveness to completely fulfill the applying merits of a new type patent, according to which the invention is thereby applied. Please examine the application carefully and grant it as a formal patent for protecting the rights of the inventor.

However, the aforementioned description is only a number of preferable embodiments according to the present invention, and not used to limit the patent scope of the invention. Thus, equivalently structural variations made to the contents of the present invention are all covered by the claims claimed thereinafter.

## Claims

1. An improved vascular clamp structure, comprising:
a clip body, arranged with a bridge part with an inflecting point, and extending a first clipping part and a second clipping part respectively from two ends of the bridge part, and making one side of the clip body forming an opening for fitting a vessel;
a first clipping piece, arranged with a first clipping body with a first fitting slot and assembled fixedly by the first fitting slot and the first clipping part of the clip body, and the first clipping body being arranged with a first groove and at least one male fastener at a face adjacent to a second clamping piece; and
a second clipping pieces, arranged with a second clipping body with a second fitting slot and assembled fixedly by the second fitting slot and the second clipping part of the clip body, and the second clipping body being arranged with a second groove and at least one female fastener matching the male fastener at another face adjacent to the first clipping piece.

2. The improved vascular clamp structure according to claim 1, wherein the clip body is set as a memory metal with the effectiveness of restoration.

3. The improved vascular clamp structure according to claim 1, wherein the first clipping part, the second clipping part, the first fitting slot and the second fitting slot are configured as wedged dovetails assembled and matching to each other.

4. The improved vascular clamp structure according to claim 3, wherein the wedged dovetails are to set the inside faces of the first fitting slot and the second fitting slot as two oblique walls and set the outside faces of the first clipping part and the second clipping part as two bevel edges corresponding to each other, and wherein bottom sides of two oblique walls are additionally arranged as a ring-guiding slot respectively to be inter-wedged fixedly with a pair of corresponding guiding rails arranged at corresponding bottom sides of two bevel edges.

5. The improved vascular clamp structure according to claim 1, wherein the first groove is set as a convex wedge block projected from the surface of the first clipping body, while the second groove is set as a concave wedge slot recessed in a surface of the second clipping body, and wherein the convex wedge block is arranged as corresponding to the configuration of the concave wedge slot.

6. The improved vascular clamp structure according to claim 5, wherein the convex wedge block is arranged with a plurality of longitudinal ribs interspaced separately, and between the longitudinal ribs, a plurality of oblique ribs are arranged as being interspaced with the longitudinal ribs, and the concave wedge slots are arranged with a plurality of longitudinal slots corresponding to the longitudinal ribs and a plurality of oblique slots corresponding to the oblique ribs.

7. The improved vascular clamp structure according to claim 1, wherein the first groove is comprised of a plurality of flat cone convex patterns projected from the surface of the first clipping body and adjacently interspaced, while the second groove is also comprised of a plurality of flat cone convex patterns projected from the surface of the second clipping body and adjacently interspaced.

8. The improved vascular clamp structure according to claim 1, wherein the male fastener is a diverging insetting piece, while the female fastener is an insetting hole with a converging structure therein.

9. The improved vascular clamp structure according to claim 8, wherein the insetting piece is comprised of two insetting ribs extending and diverging outward to a connecting side of the clipping body, and an elastic space is formed between the insetting ribs, while the converging structure is configured by forming a projection block respectively at two sides of the opening of the insetting hole, and a wedge block is formed at a center of the converging structure, making two sides of the wedge block respectively forming two holes capable of being inserted by the insetting rib.

10. The improved vascular clamp structure according to claim 8, wherein the male fastener is arranged at a center of the bottom part of the first clipping body of the first clipping piece, while the female fastener is arranged at a center of the bottom part of the second clipping body of the second clipping piece.
